# EUROPEAN PATENT SPECIFICATION

(11) **EP 1 942 875 B1**
(45) Date of publication and mention of the grant of the patent: **12.08.2015**
(21) Application number: 06842725.1
(22) Date of filing: 24.08.2006
(51) Int. Cl.: A61K 9/20

(54) **CONTROLLED RELEASE FORMULATION**
FORMULIERUNG MIT KONTROLLIERTER FREISETZUNG
FORMULATION POUR LIBERATION CONTROLEE

(30) Priority: 24.08.2005 IN MU10012005
(43) Date of publication of application: 16.07.2008
(73) Proprietor: Rubicon Research Private Limited, Mumbai 400 078 (IN)
(72) Inventor: PILGAONKAR, Pratibha, S., Mumbai 400078 (IN); RUSTOMJEE, Maharukh, T., Mumbai 400 078 (IN); GANDHI, Anilkumar, S., Mumbai 400 078 (IN); KELKAR, Atul, Mumbai 400 078 (IN); BAGDE, Pradnya, Mumbai 400 078 (IN)
(74) Representative: Atkinson, Jonathan David Mark
(86) International application number: PCT/IN2006/000309
(87) International publication number: WO 2007/052299

(56) References cited:
- WO-A-2004/012700
- WO-A-2004/093843
- WO-A-2004/108117
- WO-A-2005/065641
- US-A1- 2001 038 852
- US-A1- 2002 012 701
- US-A1- 2002 132 002
- US-A1- 2003 021 846
- US-A1- 2005 112 198
- HAMDANI, J. ET AL: "Physical and thermal characterisation of Precirol and Compritol as lipophilic glycerides for the preparation of controlled release matrix pellets" INT. J. PHARM., vol. 260, 2003, pages 47-57, XP002458082

## Description

### FIELD OF INVENTION

The present invention relates to a controlled release formulation of an active agent having high water solubility, comprising a combination of non-polymeric release retardant and pH independent non-swelling release retardant and thereby providing a controlled release of the active agent with reduced initial burst release.

The present invention particularly relates to active agents having aqueous solubility of greater than 1 mg/ml.

### BACKGROUND OF THE INVENTION

It is known in the pharmaceutical art to prepare compositions, which provide for controlled (sustained) release of pharmacologically active substances contained in the compositions after administration to humans. The advantages of sustained release formulations are well known in the pharmaceutical field. These include the ability of the given pharmaceutical preparation to maintain a desired therapeutic effect over a comparatively longer period or time, reduced side effects, etc. Moreover, for drugs having a short elimination half-life less frequent administration and better patient compliance may be obtained with sustained release preparations as compared to the conventional dosage forms.

For some active ingredients, especially some nutrients and vitamins, several other benefits are derived from such controlled release formulations. Majority of these actives have dose dependent bioavailabilities. As the dose is increased there is reduction in absorption leading to lower bioavailability. Maximum absorption of these moieties is achieved by ingestion of several spaced doses throughout the day rather than by a single large dose. Therefore sustained release forms of large doses give higher efficiency of absorption than an equivalent dose that is given in an instant release dosage form. One of such active ingredients, which could benefit from such systems, is Vitamin C and there have been several attempts in the prior art to develop its sustained release dosage forms.

Prior art discloses a large number of patents on controlled and sustained release delivery systems, many of which describe use of hydrophilic polymers such as cellulose ethers, carbomers, etc. These polymers however, fail to deliver the desired drug release characteristics for an active agent having high aqueous solubility and used in particularly high doses. The failure is particularly de to a typical release profile that is obtained with these systems, which comprises of a high initial burst release and a plateau with incomplete release towards end. High initial burst release is particularly undesirable as it leads to poor in vivo performance, and may result in dose dumping and/ or poor local tolerability due to undesirable local effects of the active agent. Several approaches have been described in prior art to tackle this limitation of hydrophilic polymers and namely use of hydrophobic polymers and waxes or coating of the dosage forms to control the initial burst release.

US patent application 2004/0175422 describes sustained release formulations using combination of ethyl cellulose and a wax for controlled release. The preparation process involved employs dispersion of drug and ethyl cellulose in a molten wax, which is then subjected to spray cooling. This process is tedious, expensive and requires special equipment. A similar combination of excipients has been employed in the controlled release formulations of tramadol in US patent application 2002/0102302.

Controlled release formulations of metformin hydrochloride are described in PCT appiication 0228181. Sustained release of the highly water soluble drug is achieved by using a combination of hydrophobic polymer and hydrophilic material. Even with use of this combination an undesirably high burst release is observed. Moreover for preparing this formulation, a special jacketed extrude-spheronizer is required and this increases both the cost and number of steps in the manufacturing process.

Use of waxes as controlled release excipients is also described in US patent 6033685 and US patent 5656296. However in both cases a complicated delivery system has been devised to achieve the desired release profile. In the case of US 6033685 an extra polymer layers is applied by compression whereas in case of US 5656296 a porous coating layer is applied.

A combination of swelling polymer and wax is discussed in US patent 2003/0021841 and US patent 5091189. Such systems work on dual principle of granulation with wax, which reduces ingress of water and prevents contact of water with the soluble active and the swelling polymer. The combination thus ensures retardation of release. However the swelling polymer requires some time before it can swell and retard drug release. This results in an undesirably high inital burst release of the active.

WO 0172286 discloses formulations in the form of beadlets showing immediate and sustained release of therapeutic material. Each beadlet comprises 1) an extruded-spheronized inner core containing modified sustained release medicament, binder and excipients; 2) an outer core comprising a fiim-forming agent and medicament for immediate release; and 3) a controlled-release coating between the inner and outer core to control the release of the inner core medicament and to provide an effective barrier between the inner core and outer layer such that any potentially disadvantageous interaction between components is minimized. Optionally, the beadlet may comprise a further coating between the inner core and the controlled-release coating that may serve to further protect the content of the inner core from exposure. The production of the beadlets is technologically demanding and requires especial spheronizing equipment and the coating process is rather lengthy-and cumbersome.

WO 04082660 describes a multi-component controlled release system that can be incorporated in oral care products, nutraceutical, food product and beverages. The controlled release system of the invention comprises solid hydrophobic nanospheres encapsulated in a moisture sensitive microspheres. The nanospheres are not individually coated by the moisture sensitive microspheres matrix, but are homogenously dispersed in the water sensitive microsphere matrix. Vinous active ingredients can be incorporated in the hydrophobic nanosphere matrix, in the water sensitive microsphere matrix, or in both the nano and micro spheres matrices. The hydrophobic material comprises natural or synthetic wax, fat, glyceride, fatty acid or its derivative. The microparticle is formed of a bioadhesive material within a moisture sensitive matrix material comprising water soluble synthetic polymer, water dispersible synthetic polymer, starch derivative, natural gum, polysaccharide, protein, hydrocolloid and mixtures thereof. This specialized drug delivery system requires equipments such as jacket mixer, homogenizer, spray dryer with a number of steps involved in manufacturing. This makes the dosage forms expensive. Further relevant prior art includes US 2001038852.

Thus, a large amount of prior art exist in the area of controlled release dosage forms. However majority of these systems show undesirable initial burst release and their performance is not independent of physiological parameters. Several approaches are also described in the prior art to overcome these limitations, but the formulation are difficult to manufacture and expensive. Hence there still exists a need for developing controlled release compositions of highly water soluble active agents, devoid of limitations discussed above and having reduced initial burst release, enhanced bioavailability and therapeutic effectiveness.

It was surprisingly found that the above desired features can be achieved by the dosage form of the present invention by employing a combination of non-polymeric release retardant and a non-swelling pH independent release retardant. It is generally known in the art that use of pH independent release retardants alone results in a high initial burst release. However its combination with non-polymeric release retardants provides a sustained release dosage form, which releases drug with reduced initial burst and at a rate that is independent of the surrounding milieu for a considerable period of time. The process employed is simple, reproducible and amenable to large scale manufacture using conventional equipment.

### OBJECT OF THE INVENTION

The object of the present invention is to prepare a controlled-release formulation of a highly water soluble active agent showing a much reduced initial burst release.

It is a further object of the present invention to provide a controlfed-release formulation of highly water soluble active agent, comprising a combination of non-polymeric release retardant and a pH independent non-swelling release retarding polymer and showing a reduced initial burst release.

Still another object of the present invention is to provide a method of manufacturing the controlled release formulation of a highly soluble active agent having desired dissolution profile with excellent physical properties.

It is yet another object of the present invention to provide a controlled-release formulation of, for example, vitamin C, comprising a combination of non-polymeric release retardant and a pH independent non-swelling release retarding polymer.

### SUMMARY OF INVENTION

According to a broad aspect of the present invention there is provide a controlled release formulation comprising:
1) therapeutically effective amount of pharmacologically active substance having high water solubility,
2) at least one non-polymeric release retardant, and
3) at least one pH independent non-swelling release retarding polymer, the active substance along with the non-polymeric release retardant is in the form of granules, wherein the granule is prepared by melt granulation.

Wherein the said dosage form provides controlled release of the active agent with reduced initial burst release.

### Brief Description of Figures:

Fig: 1: In vitro release profiles of composition 1 and 2
Fig: 2: Multimedia dissolution of vitamin C tablets of composition 2
Fig: 3: In vitro release profile of vitamin C tablets of composition 1 and 3
Fig: 4: In vitro release profiles of metoprolol succinate tablets.

### DETAILED DESCRIPTION OF THE INVENTION

The present inventors have addressed the need of developing a controlled release dosage form providing reduced initial burst release. It was surprisingly found that the much desired pH independent drug release was achieved using a combination of non-polymeric release retardant and pH independent non-swelling release retardant. Also the use of non-swelling release retardants maintains the size of the dosage form thus avoiding variability of drug release rates arising from variable transit times of the dosage form through the gastrointestinal tract.

The term 'non-swelling' as used here includes any excipient that does not swell in water or swells only moderately. This definition does not include excipients such as super disintegrants and polymers such as polyethylene oxide that swell voluminously in contact with water or aqueous media.

The term 'release retardant' as used here means any excipient that can retard the release of an active pharmaceutical ingredient and includes polymers, waxes, fatty acids etc.

A pH independent release retardant is the one whose solubility is independent of pH and hence its performance does not depend on the pH of the environment it encounters.

'Reduced initial burst release' as per this invention means the reduction in release of active ingredient from the dosage form at initial time points. The release of active ingredient in first hour according to the present invention is less than 35% and preferably less than 30%.

'High solubility' as per the invention mean active agents having a solubility of greater than 1 mg/ml.

### The Active agent

The present invention provides a sustained release formulation of a pharmacologically active agent having an aqueous solubility of greater that 1 mg/ml and may be acidic, basic or neutral in character. The invention is also applicable to active agents at different dose levels and variable solubility. The active agents may be selected from one of the following therapeutic classes that include:
anti-inflammatory, antipyretic, anticonvulsant and/or analgesic agents, tuberculostats, cardiocirculatory system drugs, antihistaminic agents, hypnotic sedatives, antineoplastic agents, antineoplastic agents, antineoplastic agents, bronchodilators, antiarrhythmic agents, surface anesthetics, antiepiteptic, synthetic adrenocortical steroids, digestive system drugs or antibiotics.

The active agents may be selected from neomycin sulphate, verapamil hydrochloride, brimonidine tartrate, morphine sulphate, lamivudine, mepivacaine hydrochloride, zidovudine, lisinopril, ropinirole hydrochloride, abacavir sulphate, pentoxifylline, valcyclovir hydrochloride, albuterol sulphate, daunorubicin, ranitidine hydrochloride, clonidine hydrochloride, ondansetron hydrochloride, diltiazem hydrochloride, acyclovir sodium, albuterol sulphate, pravastatin sodium, didanosine, atenolol, stavudine, mesalazine sodium, zanamirin, doxycycline hyclate, donepezil hydrochloride, methyldopa, timolol maleate, naloxone hydrochloride, alendronate sodium, rizatriptan beuzoate, mecamylamine hydrochloride, phenoxybenzamine hydrochloride, captopril, fluvastatin sodium, benazepril hydrochloride, alburerol sulphate, pentosan polysulphate sodium, levofloxacin,, cetirizine hydrochloride, clidaymycin phosphate, warfarin sodium, propoxyphene hydrochloride, potassium chloride, pramipexole hydrochloride metoprolol succinate, metoprolol tartrate, metformin hydrochloride, losartan potassium, methyl phenidate hydrochloride, montelukast sodium, bisoprolol fumarate, oxymorphoine hydrochloride, amantadine hydrochloride, sumatriptan succinate, tramadol hydrochloride, phenobarbital sodium, cimetidine hydrochloride, quinapril hydrochloride, levomisole hydrochloride, gabapentin, ampicillin hydrochloride, ceftrioxone sodium, mepiridine hydrochloride, guanidine hydrochloride, venlafaxine hydrochloride, propranolol hydrochloride, promethzine hydrochloride, bupropion hydrochloride, phenylephrine hydrochloride, ascorbic acid.

Preferably the active ingredient is metformin hydrochloride, metoprolol succinate, vitamin C, phenylephrine hydrochloride, bupropion hydrochloride, ropinirole hydrochloride, tramadol hydrochloride. The most preferred active ingredient is vitamin C.

The term vitamin C applies to substances that possess antiscorbutic activity. The terms vitamin C, ascorbic acid and ascorbate are commonly used interchangeably. Vitamin C is an antioxidant needed for more than 300 different metabolic functions in the body, including repair and growth of tissue, healthy gums, and adrenal gland functions. However the body cannot manufacture vitamin C, and must be obtain from the diet or in the form of supplements.

The pharmacokinetic sturdy has shown that the absorption of vitamin C depends on the amount of dietary intake. At a dietary intake of 30 milligrams daily, the vitamin is nearly completely absorbed from the lumen of the small intestine into the enterocytes. Maximum vitamin C absorption of large doses is attained by ingestion of several spaced doses throughout the day rather than by a single large dose. Further, sustained-release forms of vitamin C will give a higher efficiency of absorption than an equivalent dose that is not sustain-released. High single doses of Vitamin C quickly cause the body to reach its saturation point and not accept anymore of this essential nutrient. The rest of the vitamin is eliminated in the urine, without getting the full benefits of the dosage. Up to 90% of the dosage may be excreted. A time controlled Vitamin C system allows small amounts of Vitamin C to be released and utilized by the body over an extended period of time, allowing for more beneficial results of the vitamin. Controlled release also minimizes stomach irritation in sensitive individuals. Emanual Cheraskin and his co-workers (Ref: Int J Vit Res 1969; 39: 407-415) showed that a sustained release multivitamin formulation enables greater amounts of vitamin C to reach the tissues than a non-sustained release formula. Similarly, Taylor et al (Int J Vit Nutr Res 1977; 467: 68-74) showed that the bioavailability of vitamin C (as ascorbate) was 50% higher in a sustained release multivitamin when calculating whole blood levels, and 80% higher in plasma.

The formulation is also applicable to other forms of vitamin C such as Acerola vitamin C, Rose hip vitamin C, vitamin C with bioflavonoids, reduced acidity vitamin C, Non-acid vitamin C.

In the invention, the active agent is in the form of a granule.

In certain embodiments of the present invention, one or a combination of more than one active ingredients can also be employed.

Amount of active ingredient employed in the dosage form will be generally dependent on the therapeutically effective amount of the active ingredient. However the amount of active ingredient present in the dosage form can range from 1-80%, preferably 5-50% and more preferably 10-40% by weight of the dosage form

Controlled release of the active agent is achieved by Combining non-polymeric release retardant and pH independent non-swelling release retardant. Thus, the present invention provides for two essential components for formulating the controlled release dosage form:

### 1. Non-polymeric release retardant

The term 'release retardant' as used here includes any excipient that can retard the release of an active pharmaceutical ingredient and includes polymers, waxes, fatty acids etc. Non-polymeric release retardants are those release retardant which are not comprised of repeating units of monomers. Examples includes fatty acids, long chain alcohols, fats and oils, waxes, phospholipids, eicosonoids, terpenes, steroids.

Fatty acids are carboxylic acids derived from or contained in an animal or vegetable fat or oil. Fatty acids are composed of a chain of alkyl groups containing from 4 to 22 carbon atoms and are characterize by a terminal carboxyl group. Fatty acids useful in the present invention are selected from the group consisting of hydrogenated palm oil, hydrogenated palm kernel oil, hydrogenated peanut oil, hydrogenated rapeseed oil, hydrogenated rice bran oil, hydrogenated soybean oil, hydrogenated cottonseed oil, hydrogenated sunflower oil, hydrogenated castor oil, and the like, and mixtures thereof. Other fatty acids include, for example, decenoic acid, docosanoic acid, stearic acid, palmitic acid, lauric acid, myristic acid, and the like, and mixtures thereof. The preferred fatty acids are selected from the group consisting of hydrogenated palm oil, hydrogenated castor oil, hydrogenated cottonseed oil, stearic acid, palmitic acid, and mixtures thereof.

### Long chain monohydric alcohols such as cetyl alcohol, stearyl alcohol and mixtures thereof

Waxes are esters of fatty acids with long chain monohydric alcohols. Natural waxes are often mixtures of such esters, and may also contain hydrocarbons. Waxes are low melting organic mixtures or compounds having a high molecular weight and are solid at room temperature. Waxes may be hydrocarbons or esters of fatty acids and alcohols. Waxes useful in the present invention include natural waxes, such as animal waxes, vegetable waxes, and petroleum waxes (i.e., paraffin waxes, microcrystalline waxes, petrolatum Waxes, mineral waxes), and synthetic waxes which are edible and have a melting point within the range from about 25° C. to about 100° C. Specific examples of useful waxes are spermaceti wax, carnauba wax, Japan wax, bayberry wax, flax wax, beeswax, Chinese wax, shellac wax, lanolin wax, sugarcane wax, candelilla wax, paraffin wax, microcrystalline wax, petrolatum wax, carbowax, and the like, and mixtures thereof. Mixtures of these waxes with the fatty acids set out above may also be used.

The wax may also be a monoglyceryl ester, diglyceryl ester, or triglyceryl ester (glycerides) which is an ester formed from a fatty acid having from about 10 to about 22 carbon atoms and glycerol, wherein one or more of the hydroxyl groups of glycerol is substituted by a fatty acid. Examples of useful glycerines include glyceryl monostearate, glyceryl distearate, glyceryl tristearate, glycerol dipalmitate, glyceryl tripalmitate, glyceryl monopalmitate, glyceryl dilaurate, glyceryl trilaurate, glyceryl monolaurate, glycerol didocosanoate, glyceryl tridocosanoate, glyceryl monodocosanoate, glyceryl monocaproate, glyceryl dicaproate, glyceryl tricaproate, glyceryl monomyristate, glyceryl dimyristate, glyceryl trimyristate, glyceryl monodecenoate, glyceryl didecenoate, glycerol tridecenoate, glyceryl behenate and mixtures thereof.

In the preferred embodiment the waxes may be selected from Cutina (Hydrogenated castor oil), Hydrobase (Hydrogenated soybean oil), Castorwax (Hydrogenated castor oil), Croduret (Hydrogenated castor oil), Carbowax, Compritol (Glyceryl behenate), Sterotex (Hydrogenated cottonseed oll), Lubritab (Hydrogenated cottonseed oil), Apifil (Wax yellow), Akofine (Hydrogenated cottonseed oil), Softtisan (Hydrogenated palm oil), Hydrocote (Hydrogenated soybean oil), Corona (Lanolin), Gelucire (Macrogolglycerides Lauriques), Precirol (Glyceryl Palmitostearate), Emulcire (Cetyl alcohol), Plurol diisostearique (Polyglyceryl Diisostearate), Geleol (Glyceryl Stearate), and mixtures thereof. The most preferred non-polymeric release retardants include compritol, sterotex, lubritab, stearic acid, cetyl alcohol, and mixture thereof.

The amount of non polymeric release retardant used in the formulation may vary depending upon the medicament employed and the degree of sustained release desired. In general, the fatty acid or waxy edible material will be present in the core in an amount from about 2% to about 90%, preferably from about 5% to about 75%, and more preferably from about 5% to about 40%, by weight of the composition.

### 2. pH independent non-swelling release retardant

These are the excipients whose performance is usually independent of the pH of the environment. Majority of these are hydrophilic and swell in aqueous environment. Such release retardants due to their swelling/gelling nature may cause variability due to food effect, adhesion to the mucosa, retention in stomach etc. Therefore to reduce variabilities associated with swelling systems the present invention employs non-swelling release retardants.

Non-swelling pH independent release retardants may be selected from the sustained release excipients that includes polyvinyl alcohol, polyvinyl acetate, mixture of polyvinyl acetate (8 parts w/w) and polyvinylpyrrolidone (2 parts w/w) (Kollidon SR), Polymethacrylic acid derivatives, cellulose derivatives such as ethyl cellulose, triglycerides, waxes. Kollidon SR is the most preferred release retardant.

Kolidone SR is a sustained release matrix forming agent consisting of polyvinyl acetate (8 parts w/w) and polyvinylpyrrolidone (2 parts w/w). Kolidone SR has free-flowing, non-hygroscopic and directly compressible properties (high dry binding capacity). Further, *in vitro* drug release profiles of tablet formulations with Kolidone SR as sustained release matrix forming agent are not influenced by pH of dissolution media, ionic strength of dissolution media, speed of agitation and compression force. This unique property of Kolidone SR makes it one of the most promising sustained release matrix foming agents especially where pH independent release is desired. However Kolidone SR based formulations have tendency to show higher burst release (excessive drug release in initial hours) by dissolution of drug remaining on the tablet surface as soon as the tablet comes in contact with aqueous media/fluid, thus deviating the drug release kinetics far away from zero order kinetics (linear release). Thus, higher burst release poses major limitation on use of Kolidone SR as sustained release matrix forming agent whenever lower burst release is desired/required.

The amount of pH independent polymer used in the formulation may vary depending upon the medicament employed and the degree of sustained release. desired. In general, these polymers will be present in the formulation in an amount from about 2% to about 90%, preferably from about 5% to about 75%. and more preferably from about 5% to about 40%, by weight of the composition.

According to a preferred aspect the present invention combines the advantages of a non-polymeric release retardant (Compritol) and a pH independent polymer (Kolidon SR) to provide desired drug release profile along with minimal initial burst release. The present invention provides an oral sustained release dosage form for drugs having high water solubility.

The composition of the invention therefore typically includes pharmaceutically acceptable excipients. As is well known to those skilled in the art, pharmaceutical excipients are routinely incorporated into solid dosage forms. This is done to ease the manufacturing process as well as to improve the performance of the dosage form. Common excipients include diluents, lubricants, granulating aids, colorants, flavorants, surfactants, pH adjusters, anti-adherents and gildants etc. Such excipients are routinely used in the dosage forms of this invention.

The present invention may additionally include one or more fillers or excipients in an amount within the range of from about 0 to about 90% by weight and preferably from about 1 to about 80% by weight such as lactose, sugar, corn starch, modified corn starch, mannitol, sorbitol, inorganic salts such as calcium carbonate and/or cellulose derivatives such as wood cellulose and microcrystalline cellulose.

The binders are selected from the group comprising of starch, microcrystalline cellulose, highly dispersed silica, mannitol, lactose, polyethylene glycol, polyvinylpyrrolidone, crosslinked polyvinylpyrrolidone, polymethacryilc acid derivatives, ethyl cellulose, cross-linked carboxymethylcellulose; hydroxypropyl methyl cellulose and hydroxypropylcellulose and natural and synthetic gums. Kollidone VA 64 is the most preferred binder. Binders may be incorporated into the system at a concentration of about 0.5-20% by weight, preferably 1-10% and more preferably 2-7.5% by weight.

As the composition is in the form of a tablet, it may include one or more tableting lubricants in an amount within the range of from about 0.2 to about 8% and preferably from about 0.5 to about 2% by weight of the composition, such as magnesium stearate, stearic acid, palmitic acid, calcium stearate, talc, polyethylene glycol, colloidal silicon dioxide, sodium stearyl fumarate; carnauba wax and the like and mixtures thereof. Other conventional pharmaceutical ingredients, which may optionally be present, include preservatives, stabilizers, and FD &C colors.

The dosage form of present invention is a solid dosage form, preferably a tablet, which may vary in shape such as oval, triangle, almond, peanut, parallelogram, pentagonal, hexagonal, trapezoidal. The preferred shapes are oval and parallelogram forms.

In one of the embodiments of the present invention, the composition may optionally be coated. Surface coating may be employed for aesthetic purposes or for dimensionally stabilizing the compressed tablet. The coating may be carried out using any conventional technique employing conventional ingredients suitable for enteral use. A surface coating can for example be in the form of film using conventional polymers such as hydroxypropyl methyl cellulose, hydroxypropyl cellulose, carboxymethyl cellulose, polyvinyl alcohol poly methacrylates.

In another embodiment of the present invention, the composition may optionally be coated with a functional coat. The coat can be employed using hydrophilic polymers, hydrophobic polymers, waxes etc. either alone or in combination, along with plasticizers, colorants, opacifiers etc. The functional coat may provide a further reduction in initial burst release to achieve a near zero order drug release profile. The functional coat may also inhibit the release of active ingredient in the stomach, if so desired.

In a further illustrative embodiment, a solid pharmaceutical composition may be in the form of a multilayer system for oral administration. The system may be adapted to deliver two different active agents.

In a further illustrative embodiment a solid pharmaceutical composition in the form of a multilayer system for oral administration is adapted to deliver an active pharmaceutical agent from a first layer immediately upon reaching the gastrointestinal tract, and to deliver a further pharmaceutical agent which may be same or different from a second layer, in a controlled manner over a specific time period.

Tablets formulated according to the invention allow for controlled release active agent over at least a twelve-hour period following oral administration, the in vitro release rate preferably corresponds to the following % rate of active agent released as shown in Table 1:

**TABLE 1**

| TIME (H) | % RELEASED |
|---|---|
| 1 | 10-30 |
| 2 | 20-75 |
| 4 | 30-85 |
| 6 | 50-90 |
| 8 | 60-95 |
| 12 | 65-100 |

Yet another preferred preparation particularly suited for once-a-day dosing has an in-vitro release rate corresponding to the following % rate of active agent released as shown in Table 2:

**TABLE 2**

| TIME (H) | % RELEASED |
|---|---|
| 1 | 00-35 |
| 2 | 30-75 |
| 4 | 40-85 |
| 6 | 50-100 |
| 8 | 60-100 |
| 12 | 65-100 |

A still farther preferred preparation in accordance with the invention is also particularly suited for once-a-day dosing has an in vitro release rate corresponding to the following % rate of active agent released as shown in Table 3:

**TABLE 3**

| TIME (H) | % RELEASED |
|---|---|
| 1 | 0-30 |
| 2 | 0-40 |
| 4 | 5-55 |
| 6 | 10-65 |
| 8 | 20-75 |
| 12 | 30-90 |
| 16 | 50-100 |
| 24 | >80 |

The said dosage form as per present invention can be prepared by direct compression or granulation techniques.

Granulation is any process of size enlargement whereby small particles are gathered together into larger, permanent aggregates to render them into a freeflowing state. Melt granulation methods is used. In case of melt granulation, the non-polymeric release retardant is melted and the active agent is added and mixed with the molten mass effectively, allowed to solidify and thus producing drug granules. In another illustrative embodiment of this system, the active agent is granulated using a molten non-polymeric release retardant. In some cases, both the active agent and non-polymeric release retardant may be melted together-and cooled to room temperature.

The preparation of the tablet material is technologically simple, being limited only to technological steps that are not demanding with respect to energy and time. The method of preparation and choice of supplemental substances according to the invention, described herein, also ensure very good stability and the desired physical properties of the drug form as well as the required dissolution profile.

As the formulation is less affected by physiological variations, low patient-to-patient variability may be observed which is desirable for maximum therapeutic benefit

A controlled release preparation according to the present invention is one that achieves slow release of a drug over an extended period of time, thereby extending the duration of drug action over that achieved by conventional delivery. Preferably, such a preparation maintains a drug concentration in the blood swithin the therapeutic range for 12 hours or more.

### EXAMPLES:

### Example 1

### Preparation and the drug release profile of the sustained release Vitamin C tablet dosage form:

**Table 4: Composition of the formulation**

| **Ingredients** | **mg/tablet** | |
|---|---|---|
| | **Composition 1** | **Composition 2** |
| Vitamin C | 500.00 | 500.00 |
| Compritol | 75.00 | 100.00 |
| Kollidone SR | 125.00 | 125.00 |
| Kollidone VA 64 | 30.00 | 30.00 |
| Magnesium Stearate | 7.00 | 7.00 |
| Total weight | 737.00 | 762.00 |

### Procedure:

Weighed quantities of Vitamin C and Compritol were mixed for a period of 15 min. The blend was further mixed at about 70°C and the mass was cooled to 45°C. Thus obtained granules were sized and blended with Kollidon SR and Kollidon VA64. The blend was lubricated and compressed into tablets

In vitro release profiles of vitamin C was studied in 0.1N HCl with 1% oxalic acid using USP type I dissolution apparatus with 100 rpm rotation speed: Table 5 shows the drug release profile of Composition 1 and 2.

**Table 5: In vitro release profiles of Vitamin C:**

| **Time (Hours)** | % **released** | |
|---|---|---|
| | **Composition 1** | **Composition 2** |
| 0 | 00.00 | 00.00 |
| 1 | 31.00 | 28.68 |
| 2 | 45.40 | 41.40 |
| 4 | 65.90 | 59.16 |
| 6 | 79.3 | 69.50 |

It may be seen that the tablets of vitamin C with desired physical properties and reduced initial burst release were produced employing a simple process

### Example 2

### Multimedia dissolution of composition 2

A multimedia dissolution was carried out of tablets of composition 2 additionally in phosphate buffer pH 6.8 and acetate buffer pH 4.5 using USP type I dissolution apparatus at 100 rpm

**Table 6: Multimedia dissolution of Vitamin C of composition 2**

| **Time (hr)** | 0.1N HCl | 6.8 phosphate buffer | 4.5 acetate buffer |
|---|---|---|---|
| **0** | 0.00 | 0.00 | 0.00 |
| **1** | 28.68 | 32.20 | 29.50 |
| **2** | 41.40- | 43.30 | 41.20 |
| **4** | 59.16 | 58.40 | 59.20 |
| **6** | 69.50 | 63.50 | 73.40 |

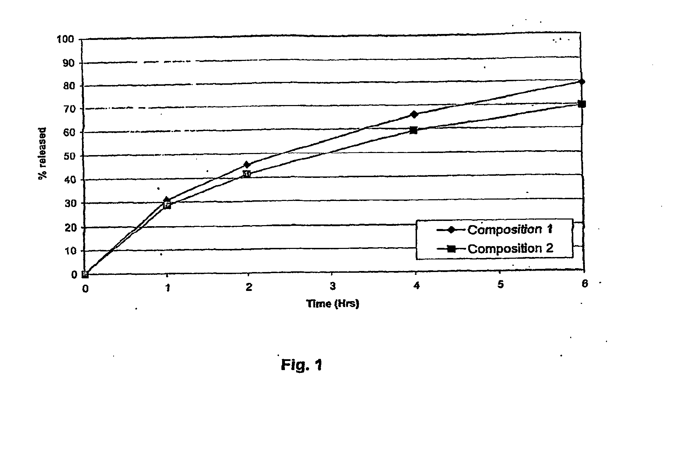

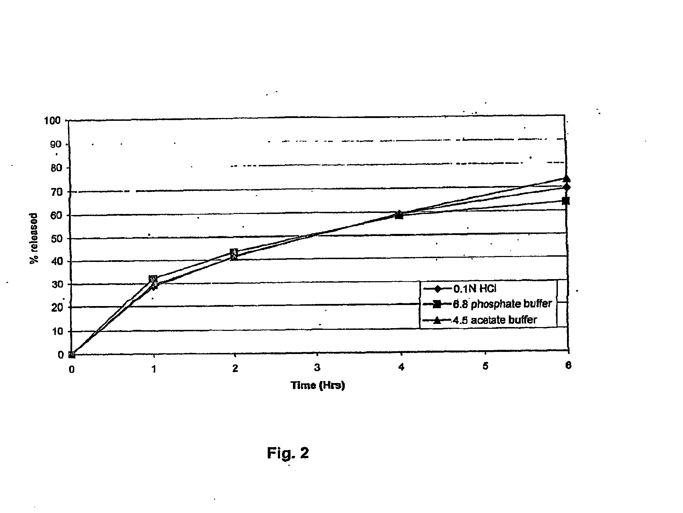

No significant difference in the in vitro release profile was, seen in the dissolution media, indicating that the composition provides a pH independent *in vitro* drug release profile for a considerable period of time (Figure 2).

### Comparative Example 3

### Preparation of the sustained release vitamin C tablet dosage form:

**Table 7: Composition of the formulation**

| **Ingredients** | **mg/tablet Composition 3** |
|---|---|
| Vitamin C | 500.00 |
| Kollidone SR | 225.00 |
| Kollidone VA 64 | 30.00 |
| Magnesium Stearate | 7.00 |
| Total weight | 662.00 |

### Procedure:

Weighed quantities of Vitamin C was blended with Kollidon SR and Kollidon VA64. The blend was lubricated and compressed into tablets

In vitro release profiles of vitamin C was studied in 0.1N HCl with 1% oxalic acid using USP type I dissolution apparatus with 100 rpm rotation speed. Table 5 shows the drug release profile of Composition 1 and 2.

**Table 8: In vitro release profiles of Vitamin C:**

| **Time (Hours)** | **% released** | |
|---|---|---|
| | **Composition 1** | **Composition 3** |
| 0 | 00.00 | 00.00 |
| 1 | 31.00 | 45.00 |
| 2 | 45.40 | 61.60 |
| 4 | 65.90 | 85.00 |
| 6 | 79.3 | 98.30 |

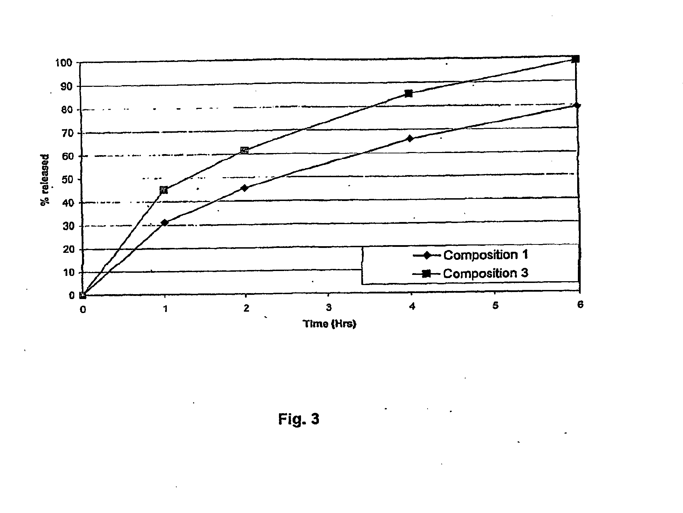

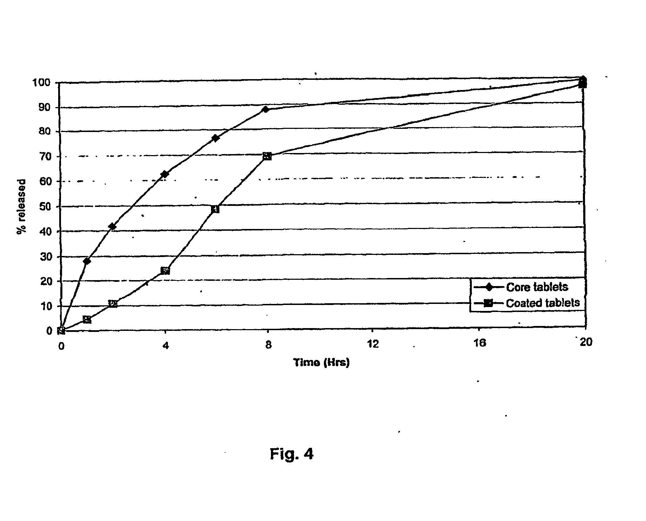

It is evident from the above examples and Figure 3, that when Kollidon SR was employed alone, the resulting release profile was much faster than the product having a combination of the two release retarding polymers (Composition 1).

Aforesaid results clearly establish the synergy existing in the combination of a non-polymeric release retardant and a non-swelling pH independent release-retardant.

### Example 4

### Preparation of the sustained release Vitamin C tablet dosage form:

**Table 9: Composition of Vitamin C tablets**

| **Ingredients** | **mg/tablet** |
|---|---|
| Vitamin C | 500.00 |
| Compritol | 45.00 |
| Eudragit RSPO | 150.00 |
| Magnesium Stearate | 5.00 |
| Total weight | 700.00 |

A weighed quantity of vitamin C was granulated using molten Compritol at slow speed. The treated Vitamin C was passed through oscillator granulator using 20-mesh sieve and blended with Eudragit RSPO, lubricated and compressed into tablets

### Example 5

### Preparation of the sustained release Vitamin C tablet dosage form:

**Table 10: Composition of Vitamin C tablets**

| **Ingredients** | **mg/tablet** |
|---|---|
| Vitamin C | 500.00 |
| Cetyl alcohol | 150.00 |
| Ethyl cellulose | 50.00 |
| Magnesium Stearate | 25.00 |
| Total weight | 725.00 |

Weighed quantities of vitamin C was granulated using molten cetyl alcohol at slow speed. Vitamin C granules were blended with ethyl cellulose, lubricated using magnesium stearate and compressed into tablets

**Table 11: In vitro release profiles of Vitamin C:**

| **Time (Hours)** | **% released** |
|---|---|
| 0 | 00.00 |
| 1 | 32.41 |
| 2 | 49.57 |
| 4 | 70.82 |
| 6 | 83.99 |
| 8 | 95.08 |

This example indicates that sustained release formulation with reduced burst release can also be obtained with a combination cetyl alcohol and ethyl cellulose.

### Comparative Example 6

### Metformin hydrochloride Extended release tablet

**Table 12: Composition of Metformin HCl tablets:**

| **Excipients** | **mg/tablet** |
|---|---|
| Metformin hydrochloride | 500 |
| Hydrogenated vegetable oil, Type I, USPNF (Lubritab) | 100 |
| Copovidone, USP (Kollidon VA 64) | 100 |
| Kollidon SR | 200 |
| *Microcrystalline* cellulose, USP (Avicel pH 102) | 80 |
| Magnesium stearate | 20 |
| **Total** | **1000** |

Metformin hydrochloride, lubritab and part of Kollidon VA 64 were dry mixed and granulated. These granules were mixed with Kollidon SR, remaining amount of Kollidon VA 64 and Avicel PH 102 and the blend thus obtained was mixed with magnesium stearate and compressed into tablets.

The tablets of metformin hydrochloride obtained showed good hardness and low friability.

### Example 7

### Venlafaxine Extended release tablet by melt granulation

**Table 13: Composition of Venlafaxine tablets:**

| **Excipients** | **mg/tablet** |
|---|---|
| Venlafaxine | 169.72 |
| Stearoyl Macrogol Glycerides, USP (Gelucire 43/01) | 100.00 |
| Kollidon SR | 300.00 |
| Lactose | 24.28 |
| Magnesium stearate | 6.00 |
| **Total** | **600.00** |

Venlafaxine was melt granulated with gelucire 3/01. The granules were mixed with Kollidon SR and Lactose DCL. The blend thus obtained was further mixed with magnesium stearate and compressed into tablets.

*In-vitro* dissolution rate studies for the tablets were carried out using USP type I apparatus at 37°C temperature and 100 rpm. 900 ml of water was employed as dissolution medium.

**Table 14: In vitro release profiles of Venlafaxine tablets:**

| **Time (Hrs)** | % **Release** |
|---|---|
| 1 | 28.8 |
| 2 | 46.1 |
| 4 | 75.0 |
| 6 | 93.7 |
| 8 | 101.2 |

Controlled release tablets of venlafaxine hydrochloride exhibited desired performance characteristics.

### Comparative Example 8

### Metoprolol succinate Extended release tablet by wet granulation

**Table 15: Composition of Metoprolol succinate tablets:**

| **Excipients** | **Mg/tablet** |
|---|---|
| Metoprolol Succinate | 47.50 |
| Hydrogenated vegetable oil, Type I, USPNF (Sterotex) | 100.00 |
| Eudragit NE 40 D | 16.00 |
| Ethyl Cellulose | 50.00 |
| Dibasic calcium phosphate | 14.60 |
| Magnesium stearate | 2.50 |
| Hypromellose 5 cps | 44.00 |
| Ethyl cellulose aqueous dispersion | 11.00 |
| Glycerin | 4.40 |
| Water, Purified | q.s. |
| **Total** | **230** |

Metoprolol succinate was mixed with sterotex and Eudragit NE 40 D and granulated. The granules were blended with ethyl cellulose and DCP and the blend was mixed with magnesium stearate and compressed into tablets. The tablets were further coated with a functional coat of ethyl cellulose and hypromellose. *In-vitro* dissolution rate studies for the tablets were carried out using USP type II apparatus at 37°C temperature and 50 rpm. 500 ml of pH 6.8 phosphate buffer was employed as dissolution medium.

**Table 16: In vitro release profiles of Metoprolol succinate:**

| **Time (Hrs)** | **% Release** | |
|---|---|---|
| | Core tablets | Coated tablets |
| 1 | 28.0 | 4.3 |
| 2 | 41.8 | 10.7 |
| 4 | 62.4 | 23.9 |
| 6 | 76.6 | 48.4 |
| 8 | 87.9 | 69.1 |
| 20 | 99.3 | 97.2 |

The tablets of metoprolol succinate exhibited desired physical and chemical characteristics. Moreover coating of tablets with ethyl cellulose and hydroxypropylmethyl cellulose further reduced the initial burst release.

### Example 9

### Metoprolol succinate Extended release tablet by melt granulation

**Table 17: Composition of Metoprolol succinate tablets:**

| **Excipients** | **mg/tablet** |
|---|---|
| Metoprolol succinate | 47.5 |
| Hydrogenated vegetable oil, Type I, USPNF (Lubritab) | 50 |
| Copovidone, USP (Kollidon VA 64) | 10 |
| Kollidon SR | 50 |
| Microcrystalline cellulose, USP (Avicel pH 102) | 60 |
| Magnesium stearate | 2.5 |
| **Total** | **220** |

Metoprolol succinate was melt granulated with lubritab and the granules were mixed with Kollidon SR, Kollidon VA 64 and MCC pH 102. The blend thus obtained was mixed with magnesium stearate and compressed into tablets.

*In-vitro* dissolution rate studies for the tablets were carried out using USP type II apparatus at 37°C temperature and 50 rpm. 500 ml of pH 6.8 phosphate buffer was employed as dissolution medium.

**Table 16: In vitro release profiles of Metoprolol succinate:**

| **Time (Hrs)** | **% Release** |
|---|---|
| 1 | 22.2 |
| 2 | 32.0 |
| 4 | 45.5 |
| 6 | 55.9 |
| 8 | 64.9 |
| 16 | 93.2 |
| 20 | 94.7 |

The tablets of metoprolol succinate exhibited desired physical and chemical characteristics.

### Comparative Example 10

### Phenylephrine hydrochloride Extended release tablet

**Table 18: Composition of phenylephrine hydrochloride tablets:**

| **Excipients** | **mg/tablet** |
|---|---|
| Phenylephrine hydrochloride | 30.0 |
| Hydrogenated vegetable oil, Type I, USPNF (Lubritab) | 25.0 |
| Starch 1500 | 10.0 |
| Kollidon SR | 75.0 |
| Microcrystalline cellulose, USP (Avicel pH 102) | 45.5 |
| Magnesium stearate | 2.5 |
| **Total** | **188.0** |

Phenylephrine hydrochloride, lubritab, Kollidon SR, Kollidon VA 64 and Avicel PH 102 were mixed together. The blend thus obtained was mixed with magnesium stearate and compressed into tablets.The tablets of phenylephrine hydrochloride obtained showed good hardness and low fiability.

## Claims

1. An oral controlled release dosage form comprising: therapeutically effective amount of at least one active substance having an aqueous solubility of greater than 1 mg/ml, at least one non-polymeric release retardant, and at least one pH independent non-swelling release retardant,
the active substance along with the non-polymeric release retardant is in the form of granules wherein the granule is prepared by melt granulation and the said dosage form provides controlled release of the active agent with reduced initial burst release.

2. The novel controlled-release oral dosage form as claimed in claim 1, wherein said pharmacologically active ingredient is selected from class of anti-inflammatory, antipyretic, anticonvulsant and/or analgesic agents, tuberculostats, cardiocirculatory system drugs, antihistaminic agents, hypnotic sedatives, antineoplastic agents, bronchodilators, antiarrhythmic agents, surface anesthetics, antiepileptic, synthetic adrenocortical steroids, digestive system drugs or antibiotics.

3. The novel controlled-release oral dosage form as claimed in claim 1, wherein said pharmacological active ingredient is selected from Neomycin Sulphate, Verapamil hydrochloride, Brimonidine tartrate, Morphine Sulphate, Lamivudine, Mepivacaine hydrochloride, Zidovudine, Lisinopril, Ropinirole hydrochloride, Abacavir sulphate, Pentoxifylline, valcyclovir hydrochloride, Albuterol Sulphate Daunorubicin, Ranitidine hydrochloride, Clonidine hydrochloride, Ondansetron hydrochloride, Diïtiazem hydrochloride, Acyclovir Sodium, Albuterol Sulphate, Pravastatin Sodium, Didanosine, Atenolol, Stavudine, Mesalazine Sodium, Zanamirin, Doxycycline hyclate, Donepezil hydrochloride, Methyldopa, Timolol maleate, Naproxen, Naloxone hydrochloride, Alendronate sodium, Rizatriptan benzoate, Mecamylamine hydrochloride, Phenoxybenzamine hydrochloride, Captopril, Fluvastatin sodium, Benazepril hydrochloride, Alburerol Sulphate, Pentosan polysulphate sodium, Levofloxacin, Cetirizine hydrochloride, Clidaymycin phosphate, Warfarin sodium, Propoxyphene hydrochloride, Potassium chloride, Pramipexole hydrochloride, Metoprolol succinate, Metoprolol tartrate, Metformin hydrochloride, Losartan potassium, Methyl phenidate hydrochloride, Montelukast sodium, Bisoprolol fumarate, Oxymorphoine hydrochloride, Amantadine hydrochloride, Sumatriptan succinate, Tramadol hydrochloride, Phenobarbital sodium, Cimetidine hydrochloride, Quinapril hydrochloride, Levomisole hydrochloride, Gabapentin, Ampicillin hydrochloride, Ceftrioxone sodium, Mepiridine hydrochloride, Guanidine hydrochloride, Venlafaxine hydrochloride, Propranolol hydrochloride, Promethzine hydrochloride, Bupropion hydrochloride, phenylephrine hydrochloride, ascorbic acid, and mixtures thereof.

4. The novel controlled-release oral dosage form as claimed in claim 3, wherein said pharmacological active ingredient is selected from metformin hydrochloride, metoprolol succinate, vitamin C, phenylephrine hydrochloride, bupropion hydrochloride, ropinirole hydrochloride, tramadol hydrochloride, and mixtures thereof. [Basis: page 8, lines 23 -24]

5. The novel controlled-release oral dosage form as claimed in claim 4, wherein said pharmacological active ingredient is vitamin C.

6. The oral controlled release dosage form according to claim 5 wherein the active substance is selected from the various forms of vitamin C such as Acerola vitamin C, Rose hip vitamin C, vitamin C with bioflavonoids, reduced acidity vitamin C and Non-acid vitamin C, and mixtures thereof.

7. The oral controlled release dosage form according to claim 1 wherein active substance is present in amounts from 1 to 80 weight %.

8. The oral controlled release dosage form according to claim 7 wherein active substance is present in amounts from 5 to 50 weight %.

9. The oral controlled release dosage form according to claim 8 wherein active substance is present in amounts from 10 to 40 weight %.

10. The oral controlled release dosage form according to claim 1 wherein the non-polymeric release retardants are selected from group of fatty acids, long chain alcohols, fats and oils, waxes, phospholipids, eicosonoids terpenes, steroids.

11. The oral controlled release dosage form according to claim 10 wherein the fatty acids are selected from the group of consisting of hydrogenated palm oil, hydrogenated palm kernel oil, hydrogenated peanut oil, hydrogenated rapeseed oil, hydrogenated rice bran oil, hydrogenated soybean oil, hydrogenated cottonseed oil, hydrogenated sunflower oil, hydrogenated castor oil, decenoic acid, docosanoic acid, stearic acid, palmitic acid, lauric acid, myristic acid, and mixtures thereof.

12. The oral controlled release dosage form according to claim 11 wherein the fatty acids are selected from the group consisting of hydrogenated palm oil, hydrogenated castor oil, hydrogenated cottonseed oil, stearic acid, palmitic acid, and mixtures thereof.

13. The oral controlled release dosage form according to claim 10 wherein long chain alcohols used are cetyl alcohol, stearyl alcohol and mixtures thereof.

14. The oral controlled release dosage form according to claim 10 wherein the waxes are spermaceti wax, carnauba wax, Japan wax, bayberry wax, flax wax, beeswax, Chinese wax, shellac wax, lanolin wax, sugarcane wax, candelilla wax, paraffin wax, microcrystalline wax, petrolatum wax, carbowax, and mixtures thereof.

15. The oral controlled release dosage form according to claim 1 the fatty acid or waxy edible material is present in the core in an amount from 2% to 90%, by weight of the composition.

16. The oral controlled release dosage form according to claim 15 the fatty acid or waxy edible material is present in the core in an amount from 5% to 75%.

17. The oral controlled release dosage form according to claim 16 the fatty acid or waxy edible material is present in the core in an amount from 5% to 40%.

18. The oral controlled release dosage form according to claim 1 wherein non- swelling pH independent release retardants is selected from polyvinyl alcohol, polyvinyl acetate, mixture of polyvinyl acetate (8 parts w/w) and polyvinylpyrrolidone (2 parts w/w) (Kollidon® SR), Polymethacrylic acid derivatives, cellulose derivatives such as ethyl cellulose, triglycerides, waxes.

19. The oral controlled release dosage form according to claim 18 wherein the most preferred non-swelling pH independent release retardant is Kollidone® SR.

20. The oral controlled release dosage form according to claim 18 wherein non- swelling pH independent release retardants is present in an amount from 2% to 90%, by weight of the composition.

21. The oral controlled release dosage form according to claim 20 wherein the non-swelling pH independent release retardants is present in an amount from 5% to 75%.

22. The oral controlled release dosage form according to claim 21 wherein the non-swelling pH independent release retardants is present in an amount from 5% to 40%.

23. The oral controlled release dosage form according to claim 1 maintains a drug concentration in the blood within the therapeutic range for 12 hours or more.

24. The novel controlled release dosage form as per claim 1 wherein the said dosage form can be tablet, capsule, pellet, granule or powder.

25. The novel controlled release dosage form as per claim 24 wherein the said dosage form is a tablet.

26. The novel controlled -release oral dosage form claimed in claim 1 further comprises binder, lubricant and diluent.

27. The novel controlled release oral dosage form as per claim 1, wherein the dosage form is prepared by wet granulation, dry granulation, melt granulation, direct compression, or molding method.

28. The novel controlled release oral dosage form as per claim 1, wherein the said dosage form is coated.

29. The novel controlled release oral dosage form as per claim 28, wherein the said coated tablet comprises coat in the form of quick dissolving film of polymer selected from the group of hydroxypropylmethyl cellulose, hydroxypropyl cellulose, carboxymethyl cellulose, polyvinyl alcohol, poly methacrylate.

30. The novel controlled release oral dosage form as per claim 29, wherein the said coat is functional coat.

31. The novel sustained release oral dosage form as per claim 30, wherein the said functional coat comprises polymer selected from the group comprising of hydrophilic polymers, hydrophobic polymers, waxes.

32. The novel sustained release oral dosage form as per claim 1, wherein the said dosage form is multilayered tablet.

33. A novel controlled-release oral dosage form comprising: therapeutically effective amount of at least one active ingredient having an aqueous solubility of greater than 1mg/ml, glyceryl behenate, and mixture of polyvinyl acetate (8 parts w/w) and polyvinylpyrrolidone (2 parts w/w), the active substance along with the glyceryl behenate is in the form of granules wherein the granule is prepared by melt granulation and the said dosage form provides controlled release of the active agent with reduced initial burst release.

34. A novel sustained-release oral dosage form comprising: therapeutically effective amount of vitamin C, glyceryl behenate, and mixture of polyvinyl acetate (8 parts w/w) and polyvinylpyrrolidone (2 parts w/w) wherein the vitamin C along with glyceryl behenate is in the form of granules wherein the granule is prepared by melt granulation and the said dosage form provides controlled release of the active agent with reduced initial burst release.

## Patentansprüche

1. Orale Darreichungsform zur kontrollierten Freisetzung, Folgendes umfassend: therapeutisch wirksame Menge von mindestens einer aktiven Substanz mit einer Wasserlöslichkeit, die 1 mg / ml übersteigt, mindestens ein nichtpolymeres Freisetzungsverzögerungsmittel, und mindestens ein pH-unabhängiges nicht quellendes Freisetzungsverzögerungsmittel, die aktive Substanz zusammen mit dem nichtpolymeren Freisetzungsverzögerungsmittel hat die Form von Granulat, wobei das Granulat durch Schmelzgranulation aufbereitet wird und die besagte Darreichungsform eine kontrollierte Freisetzung des Wirkstoffs mit reduzierter initialer Schnellfreisetzung ermöglicht.

2. Neuartige orale Darreichungsform zur kontrollierten Freisetzung wie in Anspruch 1 beansprucht, wobei der besagte pharmakologisch wirksame Stoff aus der aus entzündungshemmenden, fiebersenkenden, krampflösenden und / oder analgetischen Wirkstoffen, Tuberkulostatika, Herz-Kreislauf-Medikamenten, Antihistaminika, hypnotischen Sedativa, antineoplastischen Wirkstoffen, Bronchodilatatoren, antiarrhythmischen Wirkstoffen, Oberflächenanästhetika, Antiepileptika, synthetischen adrenokortikalen Steroiden, Medikamenten für das Verdauungssystem oder Antibiotika bestehenden Gruppe ausgewählt wird.

3. Neuartige orale Darreichungsform zur kontrollierten Freisetzung wie in Anspruch 1 beansprucht, wobei der besagte pharmakologisch wirksame Stoff aus Neomycin-Sulfat, Verapamilhydrochlorid, Brimonidintartrat, Morphinsulfat, Lamivudin, Mepivacainhydrochlorid, Zidovudin, Lisinopril, Ropinirolhydrochlorid, Abacavirsulfat, Pentoxifyllin, Valaciclovirhydrochlorid, Albuterolsulfat Daunorubicin, Ranitidinhydrochlorid, Clonidinhydrochlorid, Ondansetronhydrochlorid, Diltiazemhydrochlorid, Acyclovir-Natrium, Albuterolsulfat, Pravastatin-Natrium, Didanosin, Atenolol, Stavudin, Mesalazin-Natrium, Zanamirin, Doxycyclinhyclat, Donepezilhydrochlorid, Methyldopa, Timololmaleat, Naproxen, Naloxonhydrochlorid, Alendronat-Natrium, Rizatriptanbenzoat, Mecamylaminhydrochlorid, Phenoxybenzaminhydrochlorid, Captopril, Fluvastatin-Natrium, Benazeprilhydrochlorid, Albuterolsulfat, Pentosanpolysulfat-Natrium, Levofloxacin, Cetirizinhydrochlorid, Clindamycinphosphat, Warfarin-Natrium, Propoxyphenhydrochlorid, Kaliumchlorid, Pramipexolhydrochlorid, Metoprololsuccinat, Metoprololtartrat, Metforminhydrochlorid, Losartankalium, Methylphenidathydrochlorid, Montelukast-Natrium, Bisoprololfumarat, Oxymorphoinhydrochlorid, Amantadinhydrochlorid, Sumatriptansuccinat, Tramadolhydrochlorid, Phenobarbital-Natrium, Cimetidinhydrochlorid, Quinaprilhydrochlorid, Levomisolhydrochlorid, Gabapentin, Ampicillinhydrochlorid, Ceftriaxon-Natrium, Mepiridinhydrochlorid, Guanidinhydrochlorid, Venlafaxinhydrochlorid, Propranololhydrochlorid, Promethazinhydrochlorid, Bupropionhydrochlorid, Phenylephrinhydrochlorid, Ascorbinsäure und Mischungen davon ausgewählt wird.

4. Neuartige orale Darreichungsform zur kontrollierten Freisetzung wie in Anspruch 3 beansprucht, wobei der besagte pharmakologisch wirksame Stoff aus Metforminhydrochlorid, Metoprololsuccinat, Vitamin C, Phenylephrinhydrochlorid, Bupropionhydrochlorid, Ropinirolhydrochlorid, Tramadolhydrochlorid und Mischungen davon ausgewählt wird. [Grundlage: Seite 8, Zeilen 23-24]

5. Neuartige orale Darreichungsform zur kontrollierten Freisetzung wie in Anspruch 4 beansprucht, wobei der besagte pharmakologisch wirksame Stoff Vitamin C ist.

6. Orale Darreichungsform zur kontrollierten Freisetzung nach Anspruch 5, wobei die aktive Substanz aus den verschiedenen Formen von Vitamin C wie Acerola-Vitamin-C, Hagebutten-Vitamin-C, Vitamin C mit Bioflavonoiden, Vitamin C mit reduziertem Säuregehalt und säurefreiem Vitamin C sowie Mischungen davon ausgewählt wird.

7. Orale Darreichungsform zur kontrollierten Freisetzung nach Anspruch 1, wobei die aktive Substanz in Mengen von 1 bis 80 Gewichtsprozent vorhanden ist.

8. Orale Darreichungsform zur kontrollierten Freisetzung nach Anspruch 7, wobei die aktive Substanz in Mengen von 5 bis 50 Gewichtsprozent vorhanden ist.

9. Orale Darreichungsform zur kontrollierten Freisetzung nach Anspruch 8, wobei die aktive Substanz in Mengen von 10 bis 40 Gewichtsprozent vorhanden ist.

10. Orale Darreichungsform zur kontrollierten Freisetzung nach Anspruch 1, wobei die nichtpolymeren Freisetzungsverzögerungsmittel aus der Gruppe von Fettsäuren, langkettigen Alkoholen, Fetten und Ölen, Wachsen, Phospholipiden, Eicosanoiden, Terpenen und Steroiden ausgewählt werden.

11. Orale Darreichungsform zur kontrollierten Freisetzung nach Anspruch 10, wobei die Fettsäuren aus der aus hydriertem Palmöl, hydriertem Palmkernöl, hydriertem Erdnussöl, hydriertem Rapsöl, hydriertem Reisöl, hydriertem Sojaöl, hydriertem Baumwollsamenöl, hydriertem Sonnenblumenöl, hydriertem Rizinusöl, Decansäure, Docosansäure, Stearinsäure, Palmitinsäure, Laurinsäure, Myristinsäure und Mischungen davon bestehenden Gruppe ausgewählt werden.

12. Orale Darreichungsform zur kontrollierten Freisetzung nach Anspruch 11, wobei die Fettsäuren aus der aus hydriertem Palmöl, hydriertem Rizinusöl, hydriertem Baumwollsamenöl, Stearinsäure, Palmitinsäure und Mischungen davon bestehenden Gruppe ausgewählt werden.

13. Orale Darreichungsform zur kontrollierten Freisetzung nach Anspruch 10, wobei die langkettigen Alkohole Cetylalkohol, Stearylalkohol und Mischungen davon sind.

14. Orale Darreichungsform zur kontrollierten Freisetzung nach Anspruch 10, wobei die Wachse Spermaceti-Wachs, Carnaubawachs, Japanwachs, Wachsmyrtenwachs, Leinwachs, Bienenwachs, Chinawachs, Schellackwachs, Wollwachs, Zuckerrohrwachs, Candelillawachs, Paraffinwachs, Mikrokristallinwachs, Petrolatumwachs, Carbowachs und Mischungen davon sind.

15. Orale Darreichungsform zur kontrollierten Freisetzung nach Anspruch 1, wobei die Fettsäure oder das wachsartige essbare Material im Kern in einer Menge von 2 % bis 90 % nach Gewicht der Zusammensetzung vorhanden ist.

16. Orale Darreichungsform zur kontrollierten Freisetzung nach Anspruch 15, wobei die Fettsäure oder das wachsartige essbare Material im Kern in einer Menge von 5 % bis 75 % vorhanden ist.

17. Orale Darreichungsform zur kontrollierten Freisetzung nach Anspruch 16, wobei die Fettsäure oder das wachsartige essbare Material im Kern in einer Menge von 5 % bis 40 % vorhanden ist.

18. Orale Darreichungsform zur kontrollierten Freisetzung nach Anspruch 1, wobei die nicht quellenden pH-unabhängigen Freisetzungsverzögerungsmittel aus Polyvinylalkohol, Polyvinylacetat, einer Mischung aus Polyvinylacetat (8 Teile w/w) und Polyvinylpyrrolidon (2 Teile w/w) (Kollidon^{®} SR), Polymethacrylsäurederivate, Cellulosederivate wie Ethylcellulose, Triglyceriden und Wachsen ausgewählt werden.

19. Orale Darreichungsform zur kontrollierten Freisetzung nach Anspruch 18, wobei das bevorzugte nicht quellende pH-unabhängige Freisetzungsverzögerungsmittel Kollidon^{®} SR ist.

20. Orale Darreichungsform zur kontrollierten Freisetzung nach Anspruch 18, wobei die nicht quellenden pH-unabhängigen Freisetzungsverzögerungsmittel in einer Menge von 2 % bis 90 % nach Gewicht der Zusammensetzung vorhanden sind.

21. Orale Darreichungsform zur kontrollierten Freisetzung nach Anspruch 20, wobei die nicht quellenden pH-unabhängigen Freisetzungsverzögerungsmittel in einer Menge von 5 % bis 75 % vorhanden sind.

22. Orale Darreichungsform zur kontrollierten Freisetzung nach Anspruch 21, wobei die nicht quellenden pH-unabhängigen Freisetzungsverzögerungsmittel in einer Menge von 5 % bis 40 % vorhanden sind.

23. Orale Darreichungsform zur kontrollierten Freisetzung nach Anspruch 1 hält eine Arzneimittelkonzentration innerhalb des therapeutischen Spektrums für mindestens 12 Stunden im Blut.

24. Neuartige Darreichungsform zur kontrollierten Freisetzung nach Anspruch 1, wobei die besagte Darreichungsform eine Tablette, Kapsel, ein Kügelchen, ein Granulat oder Pulver sein kann.

25. Neuartige Darreichungsform zur kontrollierten Freisetzung nach Anspruch 24, wobei die besagte Darreichungsform eine Tablette ist.

26. Neuartige orale Darreichungsform zur kontrollierten Freisetzung wie in Anspruch 1 beansprucht, weiter umfassend Bindemittel, Gleitmittel und Verdünnungsmittel.

27. Neuartige orale Darreichungsform zur kontrollierten Freisetzung nach Anspruch 1, wobei die Darreichungsform durch Nassgranulation, Trockengranulation, Schmelzgranulation, direkte Kompression oder Formverfahren aufbereitet wird.

28. Neuartige orale Darreichungsform zur kontrollierten Freisetzung nach Anspruch 1, wobei die besagte Darreichungsform beschichtet ist.

29. Neuartige orale Darreichungsform zur kontrollierten Freisetzung nach Anspruch 28, wobei die besagte beschichtete Tablette eine Beschichtung in Form eines sich schnell auflösenden Polymerfilms, ausgewählt aus der aus Hydroxypropylmethylcellulose, Hydroxypropylcellulose, Carboxymethylcellulose, Polyvinylalkohol und Polymethacrylat bestehenden Gruppe, umfasst.

30. Neuartige orale Darreichungsform zur kontrollierten Freisetzung nach Anspruch 29, wobei die besagte Beschichtung eine funktionale Beschichtung ist.

31. Neuartige orale Darreichungsform zur verzögerten Freisetzung nach Anspruch 30, wobei die besagte funktionale Beschichtung aus der aus hydrophilen Polymeren, hydrophoben Polymeren und Wachsen bestehenden Gruppe gewähltes Polymer umfasst.

32. Neuartige orale Darreichungsform zur verzögerten Freisetzung nach Anspruch 1, wobei die besagte Darreichungsform eine mehrschichtige Tablette ist.

33. Neuartige orale Darreichungsform zur kontrollierten Freisetzung, Folgendes umfassend: therapeutisch wirksame Menge an mindestens einem wirksamen Stoff mit einer Wasserlöslichkeit, die 1 mg / ml übersteigt, Glyceryl-Behenat und eine Mischung aus Polyvinylacetat (8 Teile w/w) und Polyvinylpyrrolidon (2 Teile w/w), wobei der wirksame Stoff zusammen mit dem Glyceryl-Behenat die Form von Granulat hat und wobei das Granulat durch Schmelzgranulation aufbereitet wird und die besagte Darreichungsform eine kontrollierte Freisetzung des Wirkstoffs mit reduzierter initialer Schnellfreisetzung ermöglicht.

34. Neuartige orale Darreichungsform zur verzögerten Freisetzung, Folgendes umfassend: therapeutisch wirksame Menge an Vitamin C, Glyceryl-Behenat und einer Mischung aus Polyvinylacetat (8 Teile w/w) und Polyvinylpyrrolidon (2 Teile w/w), wobei das Vitamin C zusammen mit Glyceryl-Behenat die Form von Granulat hat, wobei das Granulat durch Schmelzgranulation aufbereitet wird und die besagte Darreichungsform eine kontrollierte Freisetzung des Wirkstoffs mit reduzierter initialer Schnellfreisetzung ermöglicht.

## Revendications

1. Forme de dosage à libération contrôlée par voie orale comprenant: une quantité thérapeutiquement efficace d'au moins une substance active ayant une solubilité dans l'eau supérieure à 1 mg/ml, au moins un retardateur de libération non polymère, et au moins un retardateur de libération non gonflant inflammable indépendant du pH, la substance active avec le retardateur de libération non polymère est sous forme de granulés, où le granulé est préparé par granulation à l'état fondu et ladite forme galénique permet une libération contrôlée de l'agent actif avec une libération de salve initiale réduite.

2. Nouvelle forme de dosage à libération contrôlée par voie orale selon la revendication 1, dans laquelle ledit ingrédient pharmacologiquement actif est choisi dans la classe des agents anti-inflammatoires, antipyrétiques, anticonvulsivant et/ou analgésiques, des tuberculostatiques, des médicaments pour le système cardiocirculatoire, des agents antihistaminiques, des sédatifs hypnotiques analgésiques, des agents antinéoplasiques, des bronchodilatateurs, des agents anti-arythmiques, des anesthésiques de surface, des antiépileptiques, des corticostéroïdes synthétiques, des médicaments du système digestif ou des antibiotiques.

3. Nouvelle forme de dosage à libération contrôlée par voie orale selon la revendication 1, dans laquelle ledit ingrédient actif pharmacologique est choisi parmi le sulfate de néomycine, le chlorhydrate de vérapamil, le tartrate de brimonidine, le sulfate de morphine, le lamivudine, le chlorhydrate de mépivacaine, la zidovudine, le lisinopril, le chlorhydrate de ropinirole, le sulfate d'abacavir, la pentoxifylline, le chlorhydrate de valcyclovir, le sulfate d' albutérol, la daunorubicine, le chlorhydrate de ranitidine, le chlorhydrate de clonidine, le chlorhydrate d'ondansétron, le chlorhydrate de ditiazem, le sodium d'acyclovir, le sulfate d'albutérol, le pravastatine sodique, la didanosine, l'aténolol, le stavudine, le sodium de mésalazine, la zanamirine, l'hyclate de doxycycline, le chlorhydrate de donépézil, la méthyldopa, le maléate de timolol, le naproxen, le chlorhydrate de naloxone, le sodium d'alendronate, le benzoate de rizatriptan, le chlorhydrate de mécamylamine, le chlorhydrate de phénoxybenzamine, le captopril, le sodium de fluvastatine, le chlorhydrate de bénazépril, le sulfate d'albutérol, le polysulfate de pentosan sodique, la lévofloxacine, le chlorhydrate de cétirizine, le phosphate de Clidaymycine, la warfarine sodique, le chlorhydrate de propoxyphène, le chlorure de potassium, le chlorhydrate de pramipexole, le succinate de métoprolol, le tartrate de métoprolol, le chlorhydrate de metformine, le potassium de Losartan, chlorhydrate de méthylphénidate, le montélukast sodique, le fumarate de bisoprolol, le chlorhydrate d'oxymorphone, le chlorhydrate d'amantadine, le succinate de sumatriptan, le chlorhydrate de tramadol, le sodium de phénobarbital, le chlorhydrate de cimétidine, le chlorhydrate de quinapril, le chlorhydrate de lévamisole, la gabapentine, le chlorhydrate d'ampicilline, le sodium de ceftriaxone, le chlorhydrate de mépiridine, le chlorhydrate de guanidine, le chlorhydrate de venlafaxine, le chlorhydrate de propranolol, le chlorhydrate de prométhazine, le chlorhydrate de bupropion, le chlorhydrate de phényléphrine, l'acide ascorbique, et des mélanges de ceux-ci.

4. Nouvelle forme de dosage à libération contrôlée par voie orale selon la revendication 3, dans laquelle ledit principe actif pharmacologique est choisi parmi le chlorhydrate de metformine, le succinate de métoprolol, la vitamine C, le chlorhydrate de phényléphrine, le chlorhydrate de bupropion, le chlorhydrate de ropinirole, le chlorhydrate de tramadol, et des mélanges de ceux-ci. [Base: page 8, lignes 23 -24]

5. Nouvelle forme de dosage à libération contrôlée par voie orale selon la revendication 4, dans laquelle ledit ingrédient pharmacologiquement actif est la vitamine C.

6. Forme de dosage à libération contrôlée par voie orale selon la revendication 5, dans laquelle la substance active est choisie parmi les différentes formes de vitamine C tels que la vitamine C d'acérola, la vitamine C de rosier muscat, la vitamine C avec bioflavonoïdes, la vitamine C d'acidité réduite et la vitamine C non-acide, et les mélanges de celles-ci.

7. Forme de dosage à libération contrôlée par voie orale selon la revendication 1, dans laquelle la substance active est présente dans des quantités de 1 à 80% en poids.

8. Forme de dosage à libération contrôlée par voie orale selon la revendication 7, dans laquelle la substance active est présente dans des quantités de 5 à 50% en poids.

9. Forme de dosage à libération contrôlée par voie orale selon la revendication 8, dans laquelle la substance active est présente dans des quantités de 10 à 40% en poids.

10. Forme de dosage à libération contrôlée par voie orale selon la revendication 1, dans laquelle les retardateurs de libération non polymères sont choisis parmi le groupe des acides gras, des alcools à longue chaîne, des graisses et des huiles, des cires, des phospholipides, des terpènes eicosanoïdes, des stéroïdes.

11. Forme de dosage à libération contrôlée par voie orale selon la revendication 10, dans laquelle les acides gras sont choisis dans le groupe constitué par l'huile de palme hydrogénée, l'huile de palmiste hydrogénée, l'huile d'arachide hydrogénée, l'huile de colza hydrogénée, l'huile de son de riz hydrogénée, l'huile de soja hydrogénée, l'huile de graine de coton hydrogénée, l'huile de tournesol hydrogénée, l'huile de ricin hydrogénée, l'acide décénoïque, l'acide docosanoïque, l'acide stéarique, l'acide palmitique, l'acide laurique, l'acide myristique et leurs mélanges.

12. Forme de dosage à libération contrôlée par voie orale selon la revendication 11, dans laquelle les acides gras sont choisis dans le groupe constitué par l'huile de palme hydrogénée, l'huile de ricin hydrogénée, l'huile de graine de coton hydrogénée, l'acide stéarique, l'acide palmitique, et leurs mélanges.

13. Forme de dosage à libération contrôlée par voie orale selon la revendication 10, dans laquelle les alcools à longue chaîne utilisés sont l'alcool cétylique, l'alcool stéarylique et leurs mélanges.

14. Forme de dosage à libération contrôlée par voie orale selon la revendication 10, dans laquelle les cires sont la cire de spermaceti, la cire de carnauba, la cire du Japon, la cire de myrique, la cire de lin, la cire d'abeille, la cire de Chine, la cire de shellac, la cire de lanoline, la cire de canne à sucre, la cire de candelilla, la cire de paraffine , la cire microcristalline, la cire de petrolatum, la carbowax, et leurs mélanges.

15. Forme de dosage à libération contrôlée par voie orale selon la revendication 1, où l'acide gras ou le matériau comestible cireux est présent dans le noyau dans une quantité de 2% à 90%, en poids de la composition.

16. Forme de dosage à libération contrôlée par voie orale selon la revendication 15, où l'acide gras ou le matériau comestible cireux est présent dans le noyau dans une quantité de 5% à 75%, en poids de la composition.

17. Forme de dosage à libération contrôlée par voie orale selon la revendication 16, où l'acide gras ou le matériau comestible cireux est présent dans le noyau dans une quantité de 5% à 40%, en poids de la composition.

18. Forme de dosage à libération contrôlée par voie orale selon la revendication 1, dans laquelle on choisit les retardateurs de libération non-gonflants indépendants de pH, parmi l'alcool polyvinylique, l'acétate de polyvinyle, un mélange d'acétate de polyvinyle (8 parties en poids/poids) et de polyvinylpyrrolidone (2 parties en poids/poids) (Kollidon® SR), les dérivés de l'acide polyméthacrylique, les dérivés de cellulose tels que la cellulose d'éthyle, les triglycérides, les cires.

19. Forme de dosage à libération contrôlée par voie orale selon la revendication 18, dans laquelle le retardateur de libération non-gonflant indépendant de pH de prédilection est le Kollidone® SR.

20. Forme de dosage à libération contrôlée par voie orale selon la revendication 18, dans laquelle les retardateurs de libération non-gonflants indépendants de pH sont présents dans une quantité comprise entre 2% et 90% en poids de la composition.

21. Forme de dosage à libération contrôlée par voie orale selon la revendication 20, dans laquelle les retardateurs de libération non-gonflants indépendants de pH sont présents dans une quantité comprise entre 5% et 75% en poids.

22. Forme de dosage à libération contrôlée par voie orale selon la revendication 21, dans laquelle les retardateurs de libération non-gonflants indépendants de pH sont présents dans une quantité comprise entre 5% et 40% en poids.

23. Forme de dosage à libération contrôlée par voie orale selon la revendication 1, maintenant une concentration de médicaments dans le sang dans la plage thérapeutiques pendant 12 heures ou plus.

24. Nouvelle forme galénique à libération contrôlée selon la revendication 1 dans laquelle ladite forme galénique peut être un comprimé, une gélule, une pastille, une granule ou une poudre.

25. Nouvelle forme galénique à libération contrôlée selon la revendication 24, où ladite forme galénique est un comprimé.

26. Nouvelle forme galénique à libération contrôlée selon la revendication 1 comprenant en outre un liant, un lubrifiant et un diluant.

27. Nouvelle forme galénique à libération contrôlée selon la revendication 1, dans laquelle la forme galénique est préparée par granulation humide, granulation sèche, granulation par fusion, compression directe ou un procédé de moulage.

28. Nouvelle forme galénique à libération contrôlée selon la revendication 1, où ladite forme galénique est enrobée.

29. Nouvelle forme galénique à libération contrôlée selon la revendication 28, dans laquelle ledit comprimé enrobé comprend un enrobage sous forme de film à dissolution rapide d'un polymère choisi dans le groupe de la cellulose hydroxypropylméthylique, la cellulose hydroxypropylique, la cellulose carboxyméthylique, l'alcool polyvinylique, le polyméthacrylate.

30. Nouvelle forme galénique à libération contrôlée selon la revendication 29, où ledit enrobage est un enrobage fonctionnel.

31. Nouvelle forme galénique à libération contrôlée selon la revendication 30, dans laquelle ledit enrobage fonctionnel comprend un polymère choisi dans le groupe comprenant des polymères hydrophiles, des polymères hydrophobes, des cires.

32. Nouvelle forme galénique à libération contrôlée selon la revendication 1, où ladite forme galénique est un comprimé multicouche.

33. Nouvelle forme galénique à libération contrôlée comprenant : une quantité thérapeutiquement efficace d'au moins un ingrédient actif ayant une solubilité dans l'eau supérieure à 1 mg/ml, le béhénate de glycéryle, et un mélange d'acétate de polyvinyle (8 parties en poids/poids) et de polyvinylpyrrolidone (2 parties en poids/poids) de la substance active avec le béhénate de glycéryle est sous la forme de granules, dans laquelle le granulé est préparé par granulation à l'état fondu et ladite forme galénique permet une libération contrôlée de l'agent actif avec libération de salve initiale réduite.

34. Nouvelle forme galénique à libération soutenue comprenant : une quantité thérapeutiquement efficace de vitamine C, de béhénate de glycéryle, et un mélange d'acétate de polyvinyle (8 parties en poids/poids) et de polyvinylpyrrolidone (2 parties en poids/poids) où la vitamine C avec le béhénate de glycéryle est sous forme de granules, dans laquelle le granulé est préparé par granulation à l'état fondu et ladite forme galénique permet une libération contrôlée de l'agent actif avec libération de salve initiale réduite.
